# EUROPEAN PATENT SPECIFICATION

(11) **EP 1 281 753 B1**
(45) Date of publication and mention of the grant of the patent: **09.12.2009**
(21) Application number: 02078114.2
(22) Date of filing: 31.07.2002
(51) Int. Cl.: C12N 1/14, C05F 9/04, C05F 11/08

(54) **Method for cultivating fungi**
Verfahren zum Kultivieren von Pilzen
Méthode de culture des champignons

(30) Priority: 01.08.2001 NL 1018680
(43) Date of publication of application: 05.02.2003
(73) Proprietor: Orgaworld B.V., 5216 PR 's-Hertogenbosch (NL)
(72) Inventor: Kaskens, Henricus Joseph Maria, 5492 CD St. Oedenrode (NL); Clarijs, Johannes Albertus Laurentius, 4623 AW Bergen op Zoom (NL)
(74) Representative: Valkonet, Rutger

(56) References cited:
- EP-A- 0 223 661
- GB-A- 2 251 250
- US-A- 4 642 131
- US-A- 4 837 155
- US-A1- 2001 049 132

## Description

The present invention relates to a method for cultivating fungi, wherein i) an inoculant material consisting of a concentrate of fungi is inoculated in a culture medium, ii) the culture medium thus inoculated is conditioned under circumstances such that said fungi multiply, and iii) the fungi formed in step ii) are harvested. The present invention furthermore relates to compost enriched with micro-organisms as well as to the use of such compost.

The method as referred to above from is known per se from US patent application US 2001/0049132, published on 6 December 2001, in which an inoculant material, comprising a concentrate of starter culture organism cells is transferred to a culture medium, wherein the cells of the starter culture organism are multiplied for a specific period of time so as to form a desired amount of cells, after which the cells thus multiplied are harvested in order to provide a starter culture. A medium substantially consisting of skimmed milk or cream is used as the culture medium. The starter culture is used for inoculating milk which is processed further for the purpose of obtaining particular milk products selected from the group of cheese, yoghurt, butter, inoculated sweet milk and a liquid fermented milk product. The use of said method for agricultural and horticultural purposes is not known from said publication.

The use of fertilisers and chemical pesticides, herbicides and fungicides in agriculture and horticulture is under dispute at present because of their harmful influence on the environment and the public health. In addition, it is common knowledge that the use of effective micro-organisms in the ground may lead to an improved soil quality and have a positive effect on the growth, the yield and the quality of crops. As a result, the use of such micro-organisms in a compost material not only leads to an enhanced organic soil fertilisation value, but in addition it has a direct positive influence on the plant. The use of such micro-organisms in a compost material increases the economic surplus value of the compost material that is enriched with micro-organisms.

Thus it is an object of the present invention to provide a method for obtaining such an alternative compost product, in which maximum concentrations of selected and naturally occurring effective micro-organisms are achieved.

Another object of the present invention is to provide a method for cultivating fungi, in which special soil fungi have been selected for their capacity to colonise plant roots, so that harmful micro-organisms will develop less easily, which has a positive effect on the plant growth.

Another object of the present invention is to provide a compost product which, in addition to the naturally occurring, bio-stimulating micro-organisms has a significant surplus value as a result of the increased presence of soil fungi.

Yet another object of the present invention is to provide a method for cultivating fungi, which fungi are incorporated in a compost product, which compost product is used in the organic cultivation of products in agriculture and horticulture.

According to the present invention, the method as referred to in the introduction is characterized in that the steps i)-ii) are carried out in a liquid phase under aerobic conditions, and in that step iii) is followed by a step iv), in which the fungi obtained in step iii) are cultivated further in a closed system under aerobic conditions in the presence of a solid carrier medium, wherein the material obtained after step iv) is mixed with natural compost, and the mixture thus obtained is used as a source of nutrients and minerals for agricultural and horticultural purposes, wherein a pure culture chosen from the group of Trichoderma harzianum, Talaromyces flavus and Arthrobotrys sp. possibly obtained by transferring a non-pure culture of Trichoderma harzianum, Talaromyces flavus and Arthrobotrys sp., respectively a number of times, is used as the inoculant material of step i).

According to the present method, the volumetric multiplication thus takes place in a number of separate steps, in which a product having a constant defined composition is obtained, which in addition meets the relevant physical, chemical and microbial quality requirements. Special embodiments are defined in the dependent claims.

The solid carrier medium that is used in the present method is preferably selected from a group of organic by-products, such as garden and kitchen waste, waste from vegetable markets and the like, and wood shavings, or a combination thereof. In a specific embodiment, it is in particular preferable to use wood shavings as the solid carrier medium, in which the wood shavings are subjected to a heat treatment prior to step iv), for example heating with hot steam at a temperature of about 102 °C for about half an hour.

The material obtained after step iv) is mixed with natural compost, and the mixture thus obtained is used as a source of nutrients and minerals for agricultural and horticultural purposes. The product obtained after said mixing with natural compost is in particular considered to be a bio-stimulating product. The advantage of using such a solid carrier material is in particular the fact that the required concentration of spores in the final product can be adjusted more easily in comparison with a conventional addition of spores in dry condition or in liquid condition. Thus the adsorption of the mycelium and the spores to the solid carrier material, preferably wood shavings, enables an optimum homogeneous mixing of the graft material in the natural compost. The advantages of the addition of bio-stimulating micro-organisms via solid natural compost compared with the known liquid or dry addition are the fact that i) a better distribution in the soil is obtained, and that ii) the microorganisms are given sufficient nutrition from the natural compost material being used, so that a sufficient amount of nutrition can reach the plant roots.

Since the step iv) of the present method is carried out in a so-called closed system, a final product which has a constant, defined composition and which meets the relevant physical, chemical and microbial quality requirements is obtained. In addition, the present method is readily controllable and it takes place in a predictable manner. In other words, the bio-stimulating fungal load of the final product can be readily and exactly adjusted.

In step iv), the temperature and the air flow are preferably controlled, whilst air is recirculated. During step iv) it is important that the Aw-value, which value defines the availability of moisture, and which is known to a person skilled in the art and thus need not be explained in more detail herein, is at least 0.90, in particular at least 0.92. If the Aw-value is lower than the above-mentioned minimum, there will be no development of fungi, or at least not to a sufficient extent. The temperature during step iv) is preferably 25-30 °C. If the Aw-value is too low, an additional amount of water will be supplied to the bed of solid carrier material. During the first stage of step i), mycelium is produced, after which the mycelium mass thus produced will start to produce spores.

In the present invention it is in particular preferable to carry out the step of mixing the material obtained after step iv) with natural compost in such a manner that the mixture that is eventually obtained has a colony forming units (cfu) (KVE-load) of at least 10⁴/g, preferably at least 10⁵-10⁶/g, and a spore concentration of at least 10⁴/g, preferably at least 10⁵-10⁶/g. Such a minimum load and spore concentration are in particular important in order to bring the fungi into contact with the roots. Furthermore it is desirable to realise conditions in each of the steps i)-iv) such that a KVE-load of 10⁷-10⁸/g and a spore concentration of 10⁷-10⁸/g are obtained in each step.

In a specific embodiment it is in particular preferable to subject the product from step iii) to a conditioning treatment between step iii) and step iv), which treatment comprises the conditioning of the product at a temperature of 3-5 °C.

The function of such a conditioning treatment is to prevent further growth of the mycelium and, in addition, prevent the spores that have been produced from developing any further. Furthermore, sporulation cannot occur, and the concentration and the vitality of the spores produced in the preceding step are maintained for a long period of time. In addition, the risk of any infections developing further is reduced. Thus it is an advantage of such a conditioning treatment that a final material exhibiting a good quality and a satisfactory fungal load for a prolonged period of time is available for the next step iv), viz. the further cultivation of the fungi obtained in step iii) in a closed system under aerobic conditions in the presence of a solid carrier material. Thus, a final material for step iv) is obtained which can be cultivated with a constant and defined quality.

In a specific embodiment, it is preferable to subject the product obtained after step iv) to a conditioning treatment, comprising conditioning of the product at a temperature of 3-5 °C in an atmosphere of CO₂ < 1% (v/v) and O₂ > 19.2% (v/v), before said mixing with natural compost takes place.

The function of such a conditioning treatment is to prevent further growth of the mycelium and, in addition, prevent the spores that have been produced from developing any further. In addition to that, the concentration and the vitality of the spores produced in the solid phase are maintained for a prolonged period of time. An increase of the carbon dioxide concentration in the air and a decrease of the oxygen concentration in the air will take place under the influence of the metabolism of the fungal biomass. Thus, the concentrations of carbon dioxide and oxygen in the air are controlled in said phase in connection with a possible toxic effect of said increased carbon dioxide concentration in the air and said decreased oxygen concentration in the air on the vitality of the fungal biomass and the fungal spores.

According to a particularly preferred embodiment, step ii) comprises at least two sub-steps iia) and iib), in which the fungi cultivated in sub-step iia) are supplied to sub-step iib), in which the fungi from sub-step iia) are further multiplied, such that the fungal biomass and the number of fungal spores from sub-step iib) are greater than the fungal biomass and the number of fungal spores from sub-step iia), which two sub-steps are carried out in a liquid phase.

According to another preferred embodiment, step ii) further comprises a third sub-step iic), in which the fungi cultivated in sub-step iib) are supplied to sub-step iic), in which the fungi from sub-step iib) are further multiplied, such that the fungal biomass and the number of fungal spores from the third sub-step iic) are greater than the fungal biomass and the number of fungal spores from sub-step iib), which sub-step iic) is carried out in a liquid phase.

In practice, step i), in which an inoculant material consisting of a concentrate of fungi is inoculated in a liquid culture medium, will yield about 20-30 ml of product, with the colony forming units (cfu) (KVE-load) preferably ranging from 10⁵ to 10⁸/g and the spore concentration ranging from 10⁵ to 10⁸/g. In practice, the material obtained in said step i) is transferred to a shaker flask, and about 400 ml of product having colony forming units (cfu) KVE-load preferably ranging from 10⁷ to 10⁸/ml and a spore concentration ranging from 10⁷ to 10⁸/ml is obtained after a period of at least 2 days, in particular about 3-4 days, under the influence of an incubation temperature of 30 °C. The product thus obtained is then transferred to a stirred tank-type bioreactor having a gross volume of about 100 l, in which the fungal material is further cultivated in a batch process for a period of at least 2 days, generally 3 to 4 days, with the colony forming units (cfu) (KVE-load) preferably ranging from 10⁷ to 10⁸/ml and the spore concentration ranging from 10⁷ to 10⁸/ml. The material that has been multiplied in such a bioreactor is subsequently transferred to a bioreactor having an even larger volume, for example a so-called bubble column having a gross volume of about 6000 1, in which an optimum oxygen supply is obtained for the aerobic metabolism of the fungus. In this process step, the temperature and the air supply are controlled and the pH value is registered. After a residence time of at least 2 days, in particular 3 to 4 days, about 4.5 m³ of product is obtained, with the colony forming units (cfu) (KVE-load) preferably ranging from 10⁷ to 10⁸/ml and the spore concentration ranging from 10⁷ to 10⁸/ml. Finally, the fungal biomass and the fungal spores are transferred to a fixed bed of carrier material in a closed system, using a batch process. The fungus uses the solid particles not only as carriers, but also as nutrient sources for its metabolism. Wood shavings form a preferred carrier material. The advantage of using a closed system is that it enables an optimum setting of the various process conditions and thus an optimum process control. In addition to that, the solid carrier material that is present in the closed system can be subjected to a heat treatment step, thus reducing the infection pressure, and enabling a better development of the grafted fungi. Moreover, the infection pressure is adequately suppressed as a result of the closed character of the system.

In the present invention it is preferred to use an organic material having a carbon content of at least 5% as the liquid culture medium, in which it is preferred to use a liquid culture medium selected from the group of molasses and starch hydrolysate. Starch hydrolysate is in particular preferred because this is a medium on which the fungi exhibit rapid growth and by means of which the largest amount of biomass is produced.

It is in particular desirable to carry out step ii) in a temperature range of 25-35 °C, with a pH value ranging from 2.5 to 9.5.

According to the invention, a pure culture of Trichoderma harzianum, possibly obtained by transferring a non-pure culture of Trichoderma harzianum a number of times, is used as the inoculant material of step i).

On the other hand, it is also possible to use a pure culture of Talaromyces flavus, possibly obtained by transferring a non-pure culture of Talaromyces flavus a number of times, as the inoculant material of step i).

In a specific embodiment it is furthermore desirable to use a pure culture of Arthrobotrys sp., possibly obtained by transferring a non-pure culture of Arthrobotrys sp. a number of times, as the inoculant material of step i).

The present invention further relates to a compost material enriched with micro-organisms obtained by using the present method.

Said mixing takes place, for example, my mixing an amount of 10 kg of product obtained after subjecting the material from step iv) to a conditioning treatment with an amount of, for example, 1000 kg of natural compost, so that a fungus and spore load of 10⁵-10⁶ KVE/g and 10⁵ -10⁶ spores/g in the final mixed product is obtained. The product thus obtained can be used as a compost product, by means of which the use of fertilizer and chemical pesticides, herbicides and fungicides can be reduced.

## Claims

1. A method for cultivating fungi, wherein i) an inoculant material consisting of a concentrate of fungi is inoculated in a culture medium, ii) the culture medium thus inoculated is conditioned under circumstances such that said fungi multiply, and iii) the fungi formed in step ii) are harvested, **characterized in that** the steps i)-ii) are carried out in a liquid phase under aerobic conditions, and **in that** step iii) is followed by a step iv), in which the fungi obtained in step iii) are cultivated further in a closed system under aerobic conditions in the presence of a solid carrier medium, wherein the material obtained after step iv) is mixed with natural compost, and the mixture thus obtained is used as a source of nutrients and minerals for agricultural and horticultural purposes, wherein a pure culture chosen from the group of Trichoderma harzianum, Talaromyces flavus and Arthrobotrys sp. possibly obtained by transferring a non-pure culture of Trichoderma harzianum, Talaromyces flavus and Arthrobotrys sp., respectively a number of times, is used as the inoculant material of step i).

2. A method according to claim 1, **characterized in that** solid carrier medium is selected from a group of organic by-products, such as garden and kitchen waste, waste from vegetable markets and the like, and wood shavings, or a combination thereof.

3. A method according to claim 2, **characterized in that** wood shavings are used as the solid carrier medium.

4. A method according to claim 3, **characterized in that** the wood shavings are subjected to a heat treatment prior to step iv).

5. A method according to claim 1, **characterized in that** the mixing step is carried out in such a manner that the mixture that is eventually obtained has colony forming units (cfu) (KVE-load) of at least 10⁴/g and a spore concentration of at least 10⁴/g.

6. A method according to claim 5, **characterized in that** the mixing step is carried out in such a manner that the mixture that is eventually obtained has colony forming units (cfu) (KVE-load) of at least 10⁵-10⁶/g and a spore concentration of at least 10⁵-10⁶/g.

7. A method according to any one or more of the preceding claims,
**characterized in that** the conditions in each of the steps i)-iv) are realised such that colony forming units (cfu) (KVE-load) of 10⁷-10⁸/g and a spore concentration of 10⁷-10⁸/g are obtained in each step.

8. A method according to any one or more of the preceding claims,
**characterized in that** the product from step iii) is subjected to a conditioning treatment between step iii) and step iv), which treatment comprises the conditioning of the product at a temperature of 3-5 °C.

9. A method according to any one or more of the preceding claims,
**characterized in that** the product obtained after step iv) is subjected to a conditioning treatment, comprising conditioning of the product at a temperature of 3-5 °C in an atmosphere of CO₂ < 1% (v/v) and O₂ > 19.2% (v/v), before said mixing with natural compost takes place.

10. A method according to any one or more of the preceding claims,
**characterized in that** step ii) comprises at least two sub-steps iia) and iib), in which the fungi cultivated in sub-step iia) are supplied to sub-step iib), in which the fungi from sub-step iia) are further multiplied, such that the fungal biomass and the number of fungal spores from sub-step iib) are greater than the fungal biomass and the number of fungal spores from sub-step iia), which two sub-steps are carried out in a liquid phase.

11. A method according to any one or more of the preceding claims,
**characterized in that** step ii) further comprises a third sub-step iic), in which the fungi cultivated in sub-step iib) are supplied to sub-step iic), in which the fungi from sub-step iib) are further multiplied, such that the fungal biomass and the number of fungal spores from the third sub-step iic) are greater than the fungal biomass and the number of fungal spores from sub-step iib), which sub-step iic) is carried out in a liquid phase.

12. A method according to any one or more of the preceding claims,
**characterized in that** the duration of step iia) is at least 2 days, the duration of step iib) is at least 2 days, the duration of step iic) is at least 2 days and the duration of step iv) is at least 15 days.

13. A method according to any one or more of the preceding claims,
**characterized in that** an organic material having a carbon content of at least 5% is used as the liquid culture medium.

14. A method according to any one or more of the preceding claims,
**characterized in that** a liquid culture medium selected from the group of molasses and starch hydrolysate is used.

15. A method according to claim 14, **characterized in that** starch hydrolysate is used as the liquid culture medium.

16. A method according to any one or more of the preceding claims,
**characterized in that** step ii) is carried out at a temperature of 25-35 °C.

17. A method according to any one or more of the preceding claims,
**characterized in that** the pH-value ranges from 2.5 to 9.5 in step ii).

18. A method according to any one or more of the preceding claims,
**characterized in that** the Aw-value is at least 0.90 during step iv).

19. Compost enriched with micro-organisms obtained by using a method according to any one or more of the claims 1-18.

20. Use of the compost of claim 19 as a biostimulation material and as a source of nutrients and minerals for agricultural and horticultural purposes.

## Patentansprüche

1. Verfahren zum Züchten von Pilzen, wobei i) ein Impfmaterial, das aus einem Pilzkonzentrat besteht, in ein Nährmedium eingeimpft wird, ii) das so beimpfte Nährmedium unter solchen Verhältnissen aufbereitet wird, dass sich die Pilze vermehren, und iii) die in Schritt ii) erzeugten Pilze geerntet werden, **dadurch gekennzeichnet, dass** die Schritte i) bis ii) in einer Flüssigphase unter aeroben Bedingungen ausgeführt werden und auf Schritt iii) ein Schritt iv) folgt, bei dem die in Schritt iii) erhaltenen Pilze in Gegenwart eines festen Trägermediums unter aeroben Bedingungen in einem geschlossenen System weitergezüchtet werden, wobei das nach iv) erhaltene Material mit natürlichem Kompost vermischt wird und die so erhaltene Mischung zu landwirtschaftlichen und gärtnerischen Zwecken als Nährstoff- und Mineralquelle verwendet wird, wobei als Impfmaterial aus Schritt i) eine Reinkultur verwendet, die aus der aus Trichoderma harzianum, Talaromyces flavus und Arthrobotrys sp. bestehenden Gruppe ausgewählt ist, die möglicherweise **dadurch** erhalten wird, dass eine nicht reine Kultur von Trichoderma harzianum, Talaromyces flavus bzw. Arthrobotrys sp. mehrere Male übertragen wird.

2. Verfahren nach Anspruch 1, **dadurch gekennzeichnet, dass** das feste Trägermedium aus einer Gruppe von organischen Nebenprodukten, wie etwa Garten- und Küchenabfällen, Abfällen von Gemüsemärkten und dergleichen und Hobelspäne, oder aus einer Kombination daraus ausgewählt ist.

3. Verfahren nach Anspruch 2, **dadurch gekennzeichnet, dass** die Hobelspäne als festes Trägermedium verwendet wird.

4. Verfahren nach Anspruch 3, **dadurch gekennzeichnet, dass** die Hobelspäne vor Schritt iv) einer Wärmebehandlung unterzogen wird.

5. Verfahren nach Anspruch 1, **dadurch gekennzeichnet, dass** der Schritt des Mischens so ausgeführt wird, dass die schließlich erhaltene Mischung koloniebildende Einheiten (cfu) (KVE-Last) von mindestens 10⁴/g und eine Sporenkonzentration von mindestens 10⁴/g hat.

6. Verfahren nach Anspruch 5, **dadurch gekennzeichnet, dass** der Schritt des Mischens so ausgeführt wird, dass die schließlich erhaltene Mischung koloniebildende Einheiten (cfu) (KVE-Last) von mindestens 10⁵-10⁶/g und eine Sporenkonzentration von mindestens 10⁵-10⁶/g hat.

7. Verfahren nach einem oder mehreren der vorhergehenden Ansprüche, **dadurch gekennzeichnet, dass** die Bedingungen in jedem der Schritte i) bis iv) so realisiert werden, dass in jedem Schritt koloniebildende Einheiten (cfu) (KVE-Last) von 10⁷-10⁸/g und eine Sporenkonzentration von 10⁷-10⁸/g erhalten werden.

8. Verfahren nach einem oder mehreren der vorhergehenden Ansprüche, **dadurch gekennzeichnet, dass** das Produkt aus Schritt iii) zwischen Schritt iii) und Schritt iv) einer Aufbereitungsbehandlung unterzogen wird, wobei die Behandlung das Aufbereiten des Produkts bei einer Temperatur von 3-5 °C umfasst.

9. Verfahren nach einem oder mehreren der vorhergehenden Ansprüche, **dadurch gekennzeichnet, dass** das nach Schritt iv) erhaltene Produkt einer Aufbereitungsbehandlung unterzogen wird, die das Aufbereiten des Produkts bei einer Temperatur von 3-5 °C in einer Atmosphäre von CO₂ < 1 % (v/v) und O₂ > 19,2% (v/v) umfasst, bevor das Mischen mit dem natürlichen Kompost durchgeführt wird.

10. Verfahren nach einem oder mehreren der vorhergehenden Ansprüche, **dadurch gekennzeichnet, dass** Schritt ii) mindestens zwei Teilschritte iia) und iib) umfasst, bei denen die in Teilschritt iia) gezüchteten Pilze Schritt iib) zugeführt werden, bei dem die Pilze aus Teilschritt iia) sich weiter vermehren, so dass die Pilzbiomasse und die Anzahl der Pilzsporen aus Teilschritt iib) die Pilzbiomasse und die Anzahl der Pilzsporen aus Teilschritt iia) übersteigen, wobei die beiden Teilschritte in einer Flüssigphase durchgeführt werden.

11. Verfahren nach einem oder mehreren der vorhergehenden Ansprüche, **dadurch gekennzeichnet, dass** Schritt ii) ferner einen dritten Teilschritt iic) umfasst, bei dem die in Teilschritt iib) gezüchteten Pilze Teilschritt iic) zugeführt werden, bei dem die Pilze aus Teilschritt iib) sich weiter vermehren, so dass die Pilzbiomasse und die Anzahl der Pilzsporen aus dem dritten Teilschritt iic) die Pilzbiomasse und die Anzahl der Pilzsporen aus Teilschritt iib) übersteigen, wobei Teilschritt iic) in einer Flüssigphase durchgeführt wird.

12. Verfahren nach einem oder mehreren der vorhergehenden Ansprüche, **dadurch gekennzeichnet, dass** die Dauer von Schritt iia) mindestens 2 Tage beträgt, die Dauer von Schritt iib) mindestens 2 Tage beträgt, die Dauer von Schritt iic) mindestens 2 Tage beträgt und die Dauer von Schritt iv) mindestens 15 Tage beträgt.

13. Verfahren nach einem oder mehreren der vorhergehenden Ansprüche, **dadurch gekennzeichnet, dass** als flüssiges Nährmedium ein organisches Material mit einem Kohlenstoffgehalt von mindestens 5 % verwendet wird.

14. Verfahren nach einem oder mehreren der vorhergehenden Ansprüche, **dadurch gekennzeichnet, dass** ein flüssiges Nährmedium verwendet wird, das aus der aus Melasse und Stärkehydrolysat bestehenden Gruppe ausgewählt ist.

15. Verfahren nach Anspruch 14, **dadurch gekennzeichnet, dass** Stärkehydrolysat als flüssiges Nährmedium verwendet wird.

16. Verfahren nach einem oder mehreren der vorhergehenden Ansprüche, **dadurch gekennzeichnet, dass** Schritt ii) bei einer Temperatur von 25-35 °C durchgeführt wird.

17. Verfahren nach einem oder mehreren der vorhergehenden Ansprüche, **dadurch gekennzeichnet, dass** der pH-Wert in Schritt ii) zwischen 2,5 und 9,5 beträgt.

18. Verfahren nach einem oder mehreren der vorhergehenden Ansprüche, **dadurch gekennzeichnet, dass** der Aw-Wert während Schritt iv) mindestens 0,90 beträgt.

19. Mit Mikroorganismen angereicherter Kompost, der unter Anwendung eines Verfahrens nach einem oder mehreren der Ansprüche 1 bis 18 erhalten wird.

20. Verwendung des Komposts aus Anspruch 19 als Biostimulationsmaterial und als Nährstoff- und Mineralquelle für landwirtschaftliche und gärtnerische Zwecke.

## Revendications

1. Procédé de culture de champignons, dans lequel i) une matière d'inoculation consistant en un concentré de champignons est inoculée dans un milieu de culture, ii) le milieu de culture ainsi inoculé est conditionné dans des conditions telles que lesdits champignons se multiplient, et iii) les champignons formés dans l'étape ii) sont collectés, **caractérisé en ce que** les étapes i) et ii) sont menées en phase liquide et en condition aérobie, et **en ce que** l'étape iii) est suivie d'une étape iv) dans laquelle les champignons obtenus dans l'étape iii) sont encore cultivés dans un système fermé en condition aérobie en présence d'un milieu de support solide, dans lequel la matière obtenue après l'étape iv) est mélangée avec du compost naturel, et le mélange ainsi obtenu est utilisé comme source de nutriments et de minéraux pour l'agriculture et l'horticulture, dans lequel une culture pure choisie dans le groupe composé de *Trichoderma harzianum, Talaromyces flavus* et *Arthrobotrys sp.,* éventuellement obtenue en transférant une culture non pure de *Trichoderma Harzianum, Talaromyces flavus* et *Arthrobotrys sp.* respectivement un certain nombre de fois, est utilisée comme matière d'inoculation de l'étape i).

2. Procédé selon la revendication 1, **caractérisé en ce que** le milieu de support solide est choisi dans le groupe des sous-produits organiques, comme des déchets de jardin et de cuisine, des déchets issus des marchés de légumes et équivalents, et des copeaux de bois, ou une de leurs combinaisons.

3. Procédé selon la revendication 2, **caractérisé en ce que** des copeaux de bois sont utilisés comme milieu de support solide.

4. Procédé selon la revendication 3, **caractérisé en ce que** les copeaux de bois sont soumis à un traitement thermique avant l'étape iv).

5. Procédé selon la revendication 1, **caractérisé en ce que** l'étape de mélange est menée de manière à ce que le mélange qui est finalement obtenu ait des unités formant colonie (ufc) (charge KVE) d'au moins 10⁴/g et une concentration en spores d'au moins 10⁴/g.

6. Procédé selon la revendication 5, **caractérisé en ce que** l'étape de mélange est menée de manière à ce que le mélange qui est finalement obtenu ait des unités formant colonie (ufc) (charge KVE) d'au moins 10⁵ à 10⁶/g et une concentration en spores d'au moins 10⁵ à 10⁶/g.

7. Procédé selon l'une quelconque ou plusieurs des revendications précédentes, **caractérisé en ce que** les conditions dans chacune des étapes i) à iv) sont réalisées de manière à ce que des unités formant colonie (ufc) (charge KVE) de 10⁷ à 10⁸/g et une concentration en spores de 10⁷ à 10⁸/g soient obtenues dans chaque étape.

8. Procédé selon l'une quelconque ou plusieurs des revendications précédentes, **caractérisé en ce que** le produit issu de l'étape iii) est soumis à un traitement de conditionnement entre l'étape iii) et l'étape iv), ledit traitement comprenant le conditionnement du produit à une température de 3-5 °C.

9. Procédé selon l'une quelconque ou plusieurs des revendications précédentes, **caractérisé en ce que** le produit obtenu après l'étape iv) est soumis à un traitement de conditionnement comprenant le conditionnement du produit à une température de 3-5 °C sous une atmosphère de CO₂ < 1 % (volume/volume) et de O₂ > 19,2 % (volume/volume) avant qu'ait lieu ledit mélange avec le compost naturel.

10. Procédé selon l'une quelconque ou plusieurs des revendications précédentes, **caractérisé en ce que** l'étape ii) comprend au moins deux sous-étapes iia) et iib), dans lesquelles les champignons cultivés dans la sous-étape iia) sont fournis à la sous-étape iib), dans laquelle les champignons de la sous-étape iia) se multiplient davantage, de sorte que la biomasse fongique et que le nombre de spores de champignons issus de la sous-étape iib) sont plus élevés que la biomasse fongique et que le nombre de spores de champignons issus de la sous-étape iia), ces deux sous-étapes étant menées en phase liquide.

11. Procédé selon l'une quelconque ou plusieurs des revendications précédentes, **caractérisé en ce que** l'étape ii) comprend en outre une troisième sous-étape iic), dans laquelle les champignons cultivés dans la sous-étape iib) sont fournis à la sous-étape iic), dans laquelle les champignons de la sous-étape iib) se multiplient davantage, de sorte que la biomasse fongique et que le nombre de spores de champignon issus de la troisième sous-étape iic) sont plus élevés que la biomasse fongique et que le nombre de spores de champignon issus de la sous-étape iib), cette sous-étape iic) étant menée en phase liquide.

12. Procédé selon l'une quelconque ou plusieurs des revendications précédentes, **caractérisé en ce que** la durée de l'étape iia) est d'au moins 2 jours, la durée de l'étape iib) est d'au moins 2 jours, la durée de l'étape iic) est d'au moins 2 jours et la durée de l'étape iv) est d'au moins 15 jours.

13. Procédé selon l'une quelconque ou plusieurs des revendications précédentes, **caractérisé en ce que** l'on utilise une matière organique ayant une teneur en carbone d'au moins 5 % est utilisée en tant que milieu de culture liquide.

14. Procédé selon l'une quelconque ou plusieurs des revendications précédentes, **caractérisé en ce qu'**un milieu de culture liquide choisi dans le groupe de la mélasse et d'un hydrolysat d'amidon.

15. Procédé selon la revendication 14, **caractérisé en ce que** l'hydrolysat d'amidon est utilisé comme milieu de culture liquide.

16. Procédé selon l'une quelconque ou plusieurs des revendications précédentes, **caractérisé en ce que** l'étape ii) est menée à une température de 25-35 °C.

17. Procédé selon l'une quelconque ou plusieurs des revendications précédentes, **caractérisé en ce que** la plage de valeurs de pH va de 2,5 à 9,5 dans l'étape ii).

18. Procédé selon l'une quelconque ou plusieurs des revendications précédentes, **caractérisé en ce que** la valeur Aw est d'au moins 0,90 pendant l'étape iv).

19. Compost enrichi avec des micro-organismes obtenus en utilisant un procédé selon l'une quelconque ou plusieurs des revendications 1 à 18.

20. Utilisation du compost selon la revendication 19 comme matière de biostimulation et comme source de nutriments et de minéraux pour l'agriculture et l'horticulture.
